# FASCICULE DE BREVET EUROPEEN

(11) **EP 0 754 449 B1**
(45) Date de publication et mention de la délivrance du brevet: **19.11.1997**
(21) Numéro de dépôt: 96401203.3
(22) Date de dépôt: 05.06.1996
(51) Int. Cl.: A61K 7/48

(54) **Composition pour lutter contre les taches et/ou le vieillissement de la peau, ses utilisations**
Hautdepigmentierungs- und/oder Alterungsschutzmittel und seine Verwendung
Skin whitening composition and/or anti-aging composition and uses thereof

(30) Priorité: 20.07.1995 FR 9508816
(43) Date de publication de la demande: 22.01.1997
(73) Titulaire: L'OREAL, 75008 Paris (FR)
(72) Inventeur: Simon, Pascal, 94400 Vitry-sur-Seine (FR); Gagnebien, Didier, 92320 Chatillon (FR)
(74) Mandataire: Lhoste, Catherine

(56) Documents cités:
- EP-A- 0 425 066
- EP-A- 0 461 827
- DE-A- 4 339 486
- FR-A- 2 680 466

## Description

L'invention se rapporte à une composition contenant des dérivés de l'acide ascorbique et de la rutine, utilisable plus spécialement dans les domaines cosmétique, dermatologique ou pharmaceutique en vue notamment de dépigmenter, prévenir et/ou lutter contre les taches et/ou le vieillissement cutanés. Cette composition peut être appliquée sur le visage, le corps, le cou, les mains et/ou les jambes d'êtres humains.

L'invention se rapporte aussi à une utilisation de cette composition pour le traitement cosmétique de la peau, à l'utilisation de cette composition pour la préparation d'une crème destinée au traitement dermatologique de la peau et à un procédé de traitement cosmétique.

Au cours du temps, il apparaît différents signes sur la peau, très caractéristiques du vieillissement, se traduisant notamment par une modification de la structure et des fonctions cutanées. Ce vieillissement est de nature physiologique mais également photo-induit, c'est-à-dire dû à une exposition répétée de la peau à la lumière solaire, notamment ultraviolette. L'action de cette lumière sur les constituants de la peau et sur le sébum sécrété par la peau entraîne en particulier la formation de radicaux libres oxygénés. Or, ces radicaux provoquent des dégats importants, notamment dans les membranes cellulaires (perméabilité des membranes), les noyaux des cellules (mutation par action sur l'ARN ou ADN), et les tissus (nécroses, dégénérescence) ; il est donc nécessaire de protéger la peau contre ces radicaux libres.

Les principaux signes cliniques du vieillissement cutané sont notamment l'apparition de ridules et de rides profondes, en augmentation avec l'âge, ainsi qu'une désorganisation du "grain" de la peau ; autrement dit, le microrelief cutané est moins régulier et présente un caractère anisotrope.

Par ailleurs, le teint de la peau est généralement modifié et apparaît plus jaune ; ceci semble être dû essentiellement à une désorganisation de la microcirculation (moins d'hémoglobine au niveau du derme papillaire). De plus, de nombreuses taches plus foncées apparaissent à la surface de la peau, et notamment sur les mains, conférant à la peau une hétérogénéité. En général, ces taches sont dues à une production importante de mélanine dans l'épiderme et/ou le derme de la peau. Dans certains cas d'exposition intense aux rayons solaires, ces taches peuvent devenir cancéreuses. Par ailleurs, il peut exister sur certaines zones de la peau des irritations diffuses et parfois des télangiectasies.

Un autre signe clinique du vieillissement est l'aspect sec et rêche de la peau qui est dû essentiellement à une desquamation plus importante ; ces squames, en diffractant les rayons lumineux, participent aussi à l'aspect un peu gris du teint.

On constate donc que les signes cliniques du vieillissement cutané résultent essentiellement d'un dysfonctionnement des principaux mécanismes biologiques intervenant au niveau de la peau.

Par ailleurs, certains peuples à peau colorée cherchent à diminuer leur couleur en utilisant des agents dépigmentants et/ou antipigmentants.

Aussi, la composition selon l'invention est une composition apte à prévenir et/ou lutter contre l'apparition du vieillissement et les signes de vieillissement existants plus spécialement photo-induits, comme les rides, les ridules, dépigmenter la peau, prévenir et/ou lutter contre les taches de pigmentation de la peau, quelle que soit leur origine, ainsi qu'à protéger la peau notamment par suppression de la formation de radicaux libres oxygénés.

Un des moyens efficace connu pour lutter contre le vieillissement prématuré de la peau consiste à apporter sur la peau des molécules capables d'aider les cellules à se défendre contre l'excès de radicaux libres photo-induits et plus spécialement des molécules présentant un puissant pouvoir réducteur hydrophile permettant notamment de réagir avec les radicaux libres commes les radicaux peroxyde, superoxyde et hydroxyle.

Une de ces molécules, capable de lutter efficacement contre ces radicaux libres et donc de renforcer les défenses du tissu cutané contre les agressions extérieures ( rayonnements ultraviolets, pollution) est l'acide ascorbique ou vitamine C du fait de ses propriété anti-oxydantes. Ce composé permet, en outre, de compenser la déficience en vitamine E, de stimuler la synthèse du tissu conjonctif et notamment du collagène de la peau ainsi que de dépigmenter la peau.

Malheureusement, en raison de sa structure chimique et de ses propriétés réductrices, l'acide ascorbique est très sensible à certains paramètres de l'environnement comme la lumière, l'oxygène, l'eau (par son pH et la présence de traces de métaux). Il s'ensuit donc une dégradation rapide de l'acide ascorbique formulé en présence de ces paramètres ainsi qu'une perte de ses propriétés, ce qui va à l'encontre de l'efficacité recherchée. Cette dégradation se traduit par l'apparition d'une couleur jaune/brun de la composition contenant cet acide.

Pour diminuer et/ou retarder la dégradation de l'acide ascorbique, on a envisagé de bloquer le site réactif de l'acide ascorbique, à savoir le site hydroxyle, par estérification avec un ose. Ainsi, on connait l'ascorbyl-2 glucoside décrit dans le document EP-A- 478404. Ce composé présente l'avantage, par rapport à la vitamine C, d'être bien plus stable. Il présente, en outre, une bonne solubilité dans l'eau et est bioconvertible en vitamine C sur la peau, grâce à certains enzymes de la peau, permettant ainsi de retrouver toutes les propriétés de la vitamine C.

Du fait de sa bonne solubilité dans l'eau, ce composé peut être incoporé dans un grand nombre d'excipients. Cependant, la demanderesse s'est aperçu que sa stabilité dans un milieu aqueux et en particulier dans une émulsion huile-dans-eau n'était bonne (supérieure à 15 jours) que si le pH était supérieur à 6. Or, la durée de stabilité des compositions cosmétiques, à température ambiante, doit être supérieure à plusieurs mois notamment parce qu'aucune date de péremption des produits n'est indiquée sur l'emballage, à l'inverse des médicaments.

Le tableau ci-après montre la stabilité au cours du temps et en fonction du pH de l'ascorbyl-2 glucoside.

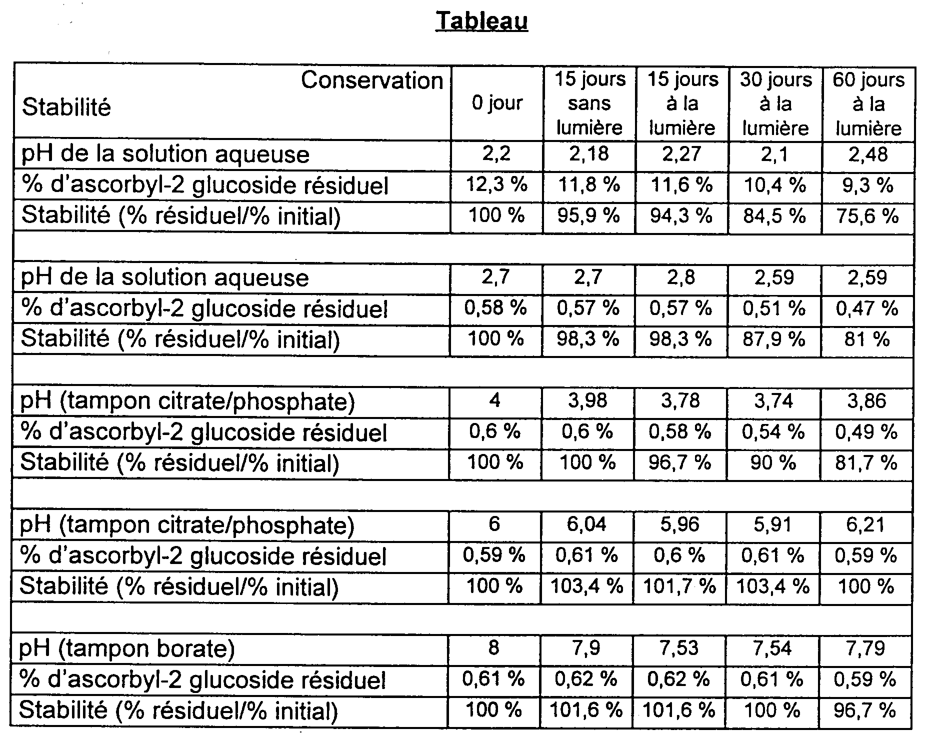

D'après ce tableau, on constate que la stabilité de l'ascorbyl-2 glucoside est améliorée pour un pH supérieur à 6. Cette stabilité à pH >6 est aussi vrai pour d'autres esters d'ose de acide ascorbique.

Comme autres molécules à propriétés anti-oxydantes, on connaît les flavonoïdes. Ces composés présentent, de plus, l'avantage d'être des anti-inflammatoires ainsi que des anti-anion superoxyde. Plus généralement, ils sont efficaces dans la désactivation des espèces radicalaires de l'oxygène. Ces propriétés sont décrites dans les documents EP-A-275005, EP-A-332478, EP-A-267155, JP-A-254250, JP-A-253394 et dans les articles " Flavonoids as antioxydants evaluated by in vitro and in situ liver chemiluminescence " de C.G. Fraga et al. , Biochemical Pharmacology, vol. 36, n°5, 1987, p.717-720 ; " Flavonoids as anti-inflammatory agents " de M.J. Alcaraz et M.J. Jimenez, Fitoterapia, vol. LIX, n°1, 1988, p.25-38 et " Flavonoids are scavengers of superoxyde anions " de J. Robak et R.J. Gryglewski, Biochemical Pharmacology, vol. 37, n°5, 1988, p.837-841.

Malheureusement, la majeure partie de ces flavonoïdes sont des composés sensibilisants capables de provoquer des allergies de contact lorsqu'ils sont appliqués sur la peau. Toutefois, la rutine est un flavonoïde qui possède un score nul dans la méthode FCA (test maximalisé à l'adjuvant de Freud comme décrit dans l'article " Aspects of the relationships between chemical structure and sensitizing potency of flavonoïds and related compounds " de H.W. Schmalle et al., Prog. Clin. Biol. Res., vol. 213, 1986, p.387-390). Cependant, la rutine est très peu soluble dans l'eau (environ 0,01 %), ce qui limite son utilisation à l'inverse des esters d'ose de la rutine tel que l'alpha-glycosyl rutine. Cette dernière est, en outre, bioconvertible au contact des enzymes de la peau et présente une meilleure stabilité à la lumière par rapport à la rutine.

En vue d'obtenir une protection efficace contre les radicaux libres ainsi que contre les rayons solaires, la demanderesse a envisagé d'associer un ester d'ose de l'acide ascorbique tel que l'ascorbyl-2 glucoside et un ester d'ose de la rutine tel que l'alpha-glucosyl rutine dans une même composition, tout en conférant à cette dernière une bonne stabilité.

Cependant, l'alpha-glycosyl rutine donne dans l'eau une solution jaune et l'intensité de la coloration dépend du pH. Si le pH est inférieur ou égal à 5, la couleur jaune est de faible intensité, de presqu'incolore à jaune pâle et stable dans le temps. Si le pH est supérieur à 5, la couleur devient de plus en plus intense et varie de jaune foncé à brun selon le pourcentage en alpha-glycosyl rutine. Dans ce cas, la composition devient inacceptable d'un point de vue cosmétique non seulement du point de vue de sa couleur mais aussi parce que celle-ci évolue dans le temps.

Autrement dit, l'ascorbyl-2 glucoside et plus généralement les esters d'ose de l'acide ascorbique sont instables pour des pH inférieurs à 5 alors que l'alpha-glycosyl rutine et plus généralement les esters d'ose de la rutine colorent de manière rédhibitoire les excipients les contenant pour des pH supérieurs à 5.

Or, la demanderesse a trouvé qu'il était possible d'obtenir une composition dans laquelle ces deux types de molécules étaient stables en choisissant un pH allant de 4 à 6. Il est tout-à-fait surprenant que l'ascorbyl-2 glucoside notamment reste stable à ce pH et que la couleur de la composition n'évolue pas dans le temps. En particulier, l'ascorbyl-2 glucoside n'est toujours pas dégradé après 12 mois à température ambiante (25° C environ) et l'alpha-glycosyl rutine reste très stable dans ce milieu car la couleur, jaune pâle, de la composition n'évolue pas.

L'invention a donc pour objet une composition stable notamment pour application topique, présentant un fort pouvoir protecteur contre les radicaux photo-induits permettant en particulier de prévenir et/ou lutter efficacement contre le vieillissement de la peau et/ou les taches cutanées, extrinsèques et intrinséques.

Selon une caractéristique essentielle de l'invention, cette composition contient une phase aqueuse ayant un pH allant de 4 à 6, au moins un ester d'ose de l'acide ascorbique et au moins un ester d'ose de la rutine.

Ainsi, de façon surprenante, la demanderesse a découvert que les dérivés d'ose de l'acide ascorbique étaient parfaitement compatibles avec les esters d'ose de la rutine et qu'ils pouvaient être formulés dans tout type d'excipient contenant de l'eau à pH 4-6. Ces dérivés présentent notamment l'avantage d'être très solubles dans l'eau et de ne pas modifier les propriétés physiques et chimiques de ces esters d'ose de la rutine.

De façon avantageuse, la composition renferme un milieu cosmétiquement et/ou dermatologiquement acceptable, c'est-à-dire un milieu compatible avec la peau y compris le cuir chevelu, les cheveux et les ongles.

La composition de l'invention peut avantageusement se présenter sous toutes les formes galéniques normalement utilisées pour une application topique, contenant de l'eau, telles que les solutions, les gels aqueux ou hydroalcooliques, les émulsions huile-dans-eau ou eau-dans-huile, et plus particulièrement les gouttelettes d'huile dispersées par des sphérules dans une phase aqueuse. Ces sphérules peuvent être des nanoparticules polymériques telles que les nanosphères et les nanocapsules ou mieux des vésicules lipidiques à lipides ioniques ou non ioniques. Ainsi, la composition de l'invention peut se présenter sous forme d'une crème, d'une pommade, d'une lotion ou d'un sérum.

La composition permet en particulier d'atténuer efficacement les rides et les ridules, de modifier le teint de la peau qui apparaît plus rosé, d'effacer les taches de pigmentation, de supprimer les squames et de donner une consistance plus élastique à la peau. Elle permet un protection efficace de la peau contre les rayons solaires ainsi qu'un blanchiment de la peau.

Les esters d'ose de l'acide ascorbique utilisables dans l'invention sont notamment les dérivés glycosylé, mannosylé, fructosylé, fucosylé, galactosylé N-acétylglucosaminé, N-acétylmuramique de l'acide ascorbique et leurs mélanges et plus spécialement l'ascorbyl-2 glucoside ou 2-O-α-D glucopyranosyl de l'acide L ascorbique ou encore le 6-O-β-D galactopyranosyl de l'acide L ascorbique. Ces derniers composés ainsi que leurs fabrications sont en particulier décrits dans les documents EP-A-487404, EP-A-425066 et J05213736.

Selon l'invention, la quantité de dérivés d'ose de l'acide ascorbique est par exemple choisie de 0,01 % à 20 % en poids par rapport au poids total de la composition et de préférence de 0,1 % à 10 %. et mieux de 0,5 % à 5 %. De plus, la composition peut contenir un ou plusieurs dérivés d'ose.

Les esters d'ose de la rutine utilisables dans l'invention sont notamment les dérivés glycosylé, mannosylé, fructosylé, fucosylé de la rutine et leurs mélanges et plus spécialement l'alpha-glycosyl rutine.

Selon l'invention, la quantité d'ester d'ose de la rutine utilisable est celle généralement utilisée dans les domaines concernés. En pratique, on utilise de 0,001 % à 5 % en poids par rapport au poids total de la composition et de préférence de 0,01 % à 1 % et mieux de 0,01 % à 0,5 %. De plus, la composition peut contenir un ou plusieurs esters d'oses de la rutine.

En vue d'améliorer la protection contre les radicaux libres, il est possible d'ajouter d'autres molécules anti-oxydantes et/ou antiradicalaires choisis parmi les chélateurs de fer, les agents antilipo-peroxyde, les composés régénérateurs de vitamine E oxydée, les agents antiradical hydroxyle, les agents anti-oxygène singulet et les agents antiradical anion superoxyde et leurs associations anisi que des filtres UVA et/ou UVB.

Les huiles utilisables dans l'invention sont celles généralement utilisées dans les domaines concernés. Elles peuvent être végétales, minérales ou synthétiques, éventuellement siliconées et/ou fluorées.

L'invention peut également contenir des adjuvants hydrophiles ou lipophiles comme des gélifiants, des conservateurs, des opacifiants, des émulsionnants, des coémulsionnants, des parfums et leurs solubilisants ou peptisants, des colorants, des pigments, des charges, ainsi que des actifs lipophiles ou hydropliles autres que les esters d'ose de l'acide ascorbique et de la rutine.

Les quantités d'huile et d'eau sont généralement celles utilisées dans les domaines considérés et sont fonction de la forme galénique de la composition. Pour une émulsion huile-dans-eau ou une dispersion d'huile dans de l'eau par des sphérules lipidiques, l'huile peut représenter de 2 % à 40 % en poids par rapport au poids total de la composition.

De même les adjuvants sont utilisés en quantité habituelle et peuvent représenter, au total, de 0,1 % à 20 % en poids. Leur quantité est fonction de leur nature.

La composition de l'invention peut être appliquée par voie topique sur toutes les parties du corps et du visage, y compris sur le cuir chevelu, les jambes et les mains.

L'invention a aussi pour objet une utilisation de la composition définie ci-dessus pour le traitement cosmétique des signes du vieillissement cutané et notamment des rides et/ou des ridules de la peau.

L'invention a encore pour objet une utilisation de la composition définie ci-dessus pour dépigmenter la peau, prévenir et/ou lutter contre les taches cutanées notamment dues au vieillissement, ainsi que pour la préparation d'une crème destinée au traitement des taches de la peau d'origine pathologique.

L'invention a encore pour objet un procédé de traitement cosmétique et/ou dermatologique de la peau, consistant à appliquer la composition définie ci-dessus sur la peau.

L'invention se rapporte aussi à l'utilisation de la composition ci-dessus pour protéger la peau contre les radicaux libres.

D'autres caractéristiques et avantages de l'invention ressortiront mieux de la description qui suit, donnée à titre illustratif. Dans les exemples ci-après de compositions cosmétiques et/ou dermatologiques conformes à l'invention, les quantités sont données en % pondéral.

### Exemple 1 : Crème huile-dans-eau pour prévenir la pigmentatation de la peau

### Composition

| | | |
|---|---|---|
| A1 | - Acide stéarique | 0,4 % |
| | - Stéarate de polyéthylène glycol (40.OE) (émulsionnant) | 3,5 % |
| | - Alcool cétylique (co-émulsionnant) | 3,2 % |
| | - Mono,di,tri-palmitostéarate de glycéryle (émulsionnant) | 3,0 % |
| | - Myristate de myristyle (huile) | 2,0 % |
| | - Palmitate d'isopropyle (huile) | 7,0 % |
| | - Iso-paraffine (6-8 moles d'isobutylène) hydrogénée (huile) | 6,5 % |
| A2 | - Cyclopentadiméthylsiloxane (huile) | 5,0 % |
| B | - Eau déminéralisée qsp | 100 % |
| | - Glycérol (hydratant) | 3,0 % |
| | - Ascorbyl-2glucoside vendu par Hayashibara | 2,0 % |
| | - Alpha- glycosyl rutine | 0,2 % |
| | - Conservateur | 0,2 % |

### Préparation de la phase A1 + A2

Les constituants de A1 sont solubilisés à 80 °C. Lorsque le mélange est limpide, la température est abaissée à 65 °C et on ajoute A2. Le mélange doit être limpide et homogène. On maintient la température de 65 °C.

### Fabrication

Dans un bécher de fabrication, les constituants de B sont solubilisés à 85 °C-90 °C. Après vérification de la limpidité, la température est ramenée à 65 °C. On réalise l'émulsion, sous agitation, en versant (A1+A2) dans B. On poursuit le refroidissement sous agitation jusqu'à la température ambiante.

On obtient une crème blanche de soin pour protéger quotidiennement la peau des méfaits du rayonnement UV et empêcher la formation des rides et ridules photo-induites.

### Exemple 2 : Gel protecteur contre les rayons solaires

### Composition

| | | |
|---|---|---|
| A | - Eau déminéralisée qsp | 100 % |
| | - Glycérine | 3,0 % |
| | - Para-hydroxybenzoate de méthyle | 0,2 % |
| | - Ascorbyl-2glucoside | 1,0 % |
| | - Alpha-glycosyl rutine | 0,05 % |
| | - Gomme de Xanthane (épaississant) | 0,2 % |
| B | - Parsol MCX (filtre UVB) | 4,0 % |
| | - Alkyl benzoate (Finsolv TN, Société Witco) | 4,0 % |
| | - Polymère carboxyvinylique alkylé (Pemulen TR 2, Société Goodrich) | 0,45 % |
| | - Triéthanolamine | 0,45 % |

### Fabrication

On prépare la phase A en saupoudrant, sous agitation le gélifiant dans l'eau contenant les ingrédients dissouts. On émulsionne en incorporant la phase B à la phase A, sous forte agitation. On lisse et on laisse refroidir sous agitation à pales lentesjusqu'à la température ambiante. La fabrication du gel est terminée.

### Exemple 3 : Lotion " teint clair "

### Composition

| | | |
|---|---|---|
| A | - Triglycérides ricinoléiques hydrogénés oxyéthylénés (60 OE) (peptisant) | 0,09 % |
| | - Parfum | 0,03 % |
| B | - Eau déminéralisée qsp | 100 % |
| | - Ascorbyl-2 glucoside | 1,0 % |
| | - Alpha-glycosyl rutine | 0,05 % |
| | - Conservateur | 0,3 % |

### Fabrication

On mélange les constituants de A à 40 °C. Lorsqu'ils sont parfaitement solubilisés, on ajoute successivement les constituants de B à température ambiante. On maintient l'agitation et on vérifie la bonne solubilisation des constituants ; le mélange doit être limpide. La fabrication est terminée.

On obtient une lotion limpide pour empêcher et diminuer la pigmentation de la peau.

## Revendications

1. Composition contenant une phase aqueuse, caractérisée en ce qu'elle contient, en outre, au moins un ester d'ose de la rutine et au moins un ester d'ose de l'acide ascorbique et en ce que la phase aqueuse a un pH allant de 4 à 6.

2. Composition selon la revendication 1, caractérisée en ce que l'ester d'ose de la rutine est l'alpha-glycosyl rutine.

3. Composition selon la revendication 1 ou 2, caractérisée en ce que l'ester d'ose de l'acide ascorbique est l'ascorbyl-2-glucoside.

4. Composition selon l'une des revendications précédentes, caractérisée en ce qu'elle se présente sous forme d'une émulsion huile-dans-eau ou sous forme d'une dispersion de sphérules lipidiques.

5. Composition selon l'une des revendications précédentes, caractérisée en ce que l'ester d'ose de l'acide ascorbique représente de 0,01 % à 20 % en poids par rapport au poids total de la composition.

6. Composition selon l'une des revendications précédentes, caractérisée en ce que l'ester d'ose de la rutine représente de 0,001 % à 5 % en poids par rapport au poids total de la composition.

7. Composition selon l'une des revendications précédentes, caractérisée en ce qu'elle contient, de plus, des adjuvants hydrophiles ou lipophiles.

8. Composition selon la revendication précédente, caractérisée en ce que les adjuvants sont choisis parmi les gélifiants, les conservateurs, les parfums, les charges, les matières colorantes, les actifs autres que l'ester d'ose de l'acide ascorbique et l'ester d'ose de la rutine.

9. Composition selon l'une des revendications précédentes, caractérisée en ce qu'elle contient un milieu cosmétiquement et/ou dermatologiquement acceptable.

10. Utilisation de la composition selon l'une des revendications précédentes pour protéger la peau contre les radicaux libres.

11. Utilisation de la composition selon l'une des revendications 1 à 9 pour prévenir et/ou lutter contre les signes du vieillissement cutané.

12. Utilisation de la composition selon l'une des revendications 1 à 9 pour dépigmenter la peau, prévenir et/ou lutter contre les taches cutanées, les rides et/ou les ridules de la peau.

13. Utilisation de la composition selon l'une des revendications 1 à 9 pour préparer une crème destinée au traitement des maladies de peau conférant à cette dernière des taches cutanées.

14. Procédé de traitement cosmétique de la peau, caractérisé en ce qu'il consiste à appliquer sur la peau une composition selon l'une des revendications 1à 9.

## Claims

1. Composition containing an aqueous phase, characterized in that it also contains at least one saccharide ester of rutin and at least one saccharide ester of ascorbic acid and in that the aqueous phase has a pH in the range from 4 to 6.

2. Composition according to Claim 1, characterized in that the saccharide ester of rutin is alpha-glycosyl rutin.

3. Composition according to Claim 1 or 2, characterized in that the saccharide ester of ascorbic acid is ascorbyl-2-glucoside.

4. Composition according to one of the preceding claims, characterized in that it is in the form of an oil-in-water emulsion or in the form of a dispersion of lipid spherules.

5. Composition according to one of the preceding claims, characterized in that the saccharide ester of ascorbic acid represents from 0.01 % to 20 % by weight relative to the total weight of the composition.

6. Composition according to one of the preceding claims, characterized in that the saccharide ester of rutin represents from 0.001 % to 5 % by weight relative to the total weight of the composition.

7. Composition according to one of the preceding claims, characterized in that it also contains hydrophilic or lipophilic adjuvants.

8. Composition according to the preceding claim, characterized in that the adjuvants are chosen from gelling agents, preserving agents, fragrances, fillers, dyestuffs and active agents other than the saccharide ester of ascorbic acid and the saccharide ester of rutin.

9. Composition according to one of the preceding claims, characterized in that it contains a cosmetically and/or dermatologically acceptable medium.

10. Use of the composition according to one of the preceding claims for protecting the skin against free radicals.

11. Use of the composition according to one of Claims 1 to 9 for preventing and/or combating the signs of ageing of the skin.

12. Use of the composition according to one of Claims 1 to 9 for depigmenting the skin and preventing and/or combating skin blemishes and wrinkles and/or fine lines on the skin.

13. Use of the composition according to one of Claims 1 to 9 to prepare a cream for treating skin diseases which impart blemishes to the skin.

14. Process for the cosmetic treatment of the skin, characterized in that it consists in applying a composition according to one of Claims 1 to 9 to the skin.

## Patentansprüche

1. Zusammensetzung, enthaltend eine wäßrige Phase, dadurch gekennzeichnet, daß sie ferner mindestens einen Saccharidester von Rutin und mindestens einen Saccharidester von Ascorbinsäure enthalt und daß die wäßrige Phase einen pH-Wert von 4-6 aufweist.

2. Zusammensetzung nach Anspruch 1, dadurch gekennzeichnet, daß es sich beim Saccharidester von Rutin um alpha-Glycosylrutin handelt.

3. Zusammensetzung nach Anspruch 1 oder 2, dadurch gekennzeichnet, daß es sich beim Saccharidester von Ascorbinsäure um 2-Ascorbylglucosid handelt.

4. Zusammensetzung nach einem der vorstehenden Ansprüche, dadurch gekennzeichnet, daß sie in Form einer Ölin-Wasser-Emulsion oder in Form einer Dispersion von Lipidkügelchen vorliegt.

5. Zusammensetzung nach einem der vorstehenden Ansprüche, dadurch gekennzeichnet, daß der Saccharidester von Ascorbinsäure 0,01 bis 20 Gew.-%, bezogen auf das Gesamtgewicht der Zusammensetzung, ausmacht.

6. Zusammensetzung nach einem der vorstehenden Ansprüche, dadurch gekennzeichnet, daß der Saccharidester von Rutin 0,001 bis 5 Gew.-%, bezogen auf das Gesamtgewicht der Zusammensetzung, ausmacht.

7. Zusammensetzung nach einem der vorstehenden Ansprüche, dadurch gekennzeichnet, daß sie ferner hydrophile oder lipophile Hilfsstoffe enthält.

8. Zusammensetzung nach dem vorstehenden Anspruch, dadurch gekennzeichnet, daß sie Hilfsstoffe enthält, die unter gelbildenden Mitteln, Konservierungsmitteln, Parfums, Füllstoffen, Färbemitteln und anderen Wirkstoffen, die von Saccharidestern von Ascorbinsäure und Saccharidestern von Rutin abweichen, ausgewählt sind.

9. Zusammensetzung nach einem der vorstehenden Ansprüche, dadurch gekennzeichnet, daß sie ein kosmetisch und/oder dermatologisch verträgliches Medium enthält.

10. Verwendung der Zusammensetzung nach einem der vorstehenden Ansprüche zum Schutz der Haut gegen freie Radikale.

11. Verwendung der Zusammensetzung nach einem der Ansprüche 1 bis 9 zur Vorbeugung und/oder Bekämpfung von Alterungserscheinungen der Haut.

12. Verwendung der Zusammensetzung nach einem der Ansprüche 1 bis 9 zur Depigmentierung der Haut und zur Vorbeugung und/oder Bekämpfung von Flecken, Falten und/oder Fältchen der Haut.

13. Verwendung der Zusammensetzung nach einem der Ansprüche 1 bis 9 zur Herstellung einer Creme, die zur Behandlung von Hautkrankheiten, die Hautflecken verursachen, bestimmt ist.

14. Verfahren zur kosmetischen Behandlung der Haut, dadurch gekennzeichnet, daß es im Aufbringen einer Zusammensetzung nach einem der Ansprüche 1 bis 9 auf die Haut besteht.
